# EUROPEAN PATENT SPECIFICATION

(11) **EP 1 260 240 B1**
(45) Date of publication and mention of the grant of the patent: **13.04.2005**
(21) Application number: 02076920.4
(22) Date of filing: 16.05.2002
(51) Int. Cl.: A61M 5/14, A61M 5/168, G01F 1/704

(54) **Device for measuring and controlling a liquid flow.**
Vorrichtung zur Messung und zur Kontrolle von Flüssigkeitsfluss
Dispositif de mesure et de contrôle de débit

(30) Priority: 23.05.2001 NL 1018148
(43) Date of publication of application: 27.11.2002
(73) Proprietor: ROELOFS OCTROOIEN EN INVESTERINGEN B.V., 5628 NM Eindhoven (NL)
(72) Inventor: Roelofs, Bernardus Johannes Gerardus Maria, 5628 NM EIndhoven (NL)
(74) Representative: Valkonet, Rutger

(56) References cited:
- EP-A- 0 105 989
- EP-A- 0 120 098
- DE-A- 19 600 667
- FR-A- 2 767 874
- US-A- 3 066 853
- US-A- 3 329 390
- US-A- 3 826 137
- US-A- 4 432 761

## Description

The invention relates to a device for measuring a liquid flow through a tube, which tube, which has an inflow side and an outflow side disposed under said inflow side, and is filled with said liquid, as a result of which a liquid column is formed in the tube, with human senses or sensors being used for measuring the liquid flow, and as well as applications therefor.

### Prior art

From EP 0 120 098 a devi ce is s known having a conduit for delivering a continuous liquid flow. Said conduit is provided with a longitudinally extending slit, serving as a means for breaking the liquid column flowing through the conduit in segments, wherein the liquid segments and air filled segments in between have an equal length. Hence, in EP 0 120 098 the liquid flow can not be controlled, but has a continuous non changing nature.

### Object of the invention

The invention has been made with a view to eliminating the above limitations by providing new measuring methods and apparatuses which result in effective, inexpensive, sensitive, real-time systems such as medical infusion systems, urine-trend indicators or metering devices in soft drink dispensers.

### Liquid flow measurement of a downward flow in a tube which is open at the bottom side

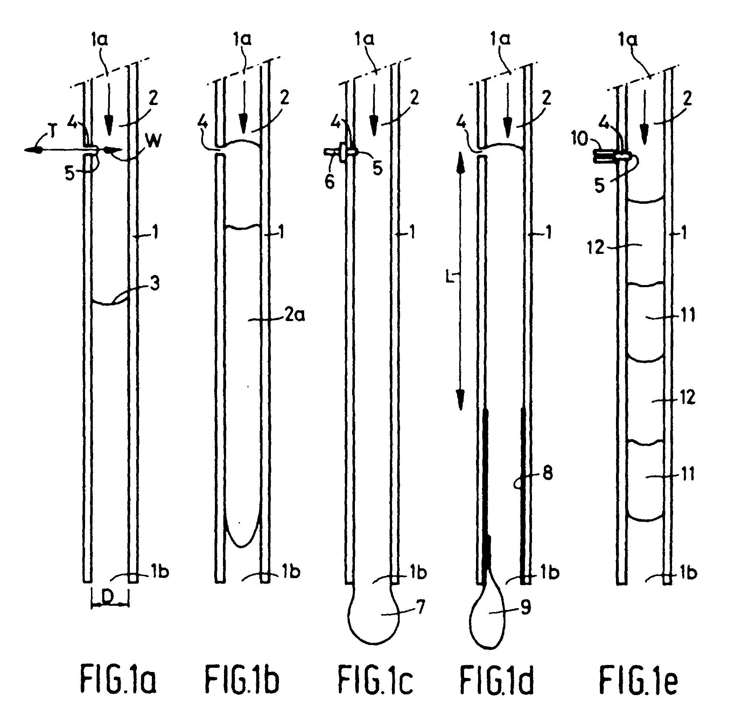

In a device as described in the first paragraph, an embodiment according to the invention of which will be explained below with reference to the appended Figures 1a -1e, the measuring of the change in length of the growing liquid column in time is a measure of the liquid flow. As a result of said growing, the meniscus level of the liquid will move in the direction of the outflow side of the tube. The change in the length of the liquid column and in the position of the meniscus, measured in time, is a measure of the liquid flow. All this is shown in Figure 1a. A suitable selection of the materials of the liquid 2 and the tube 1 having an inflow opening 1a and an outflow opening 1b prevents the liquid from draining off in a thin layer along the tube wall. In addition to that, the diameter of the tube and the properties of the material of the tube wall are such that the liquid is not retained in the tube by capillary action. It is the environmental pressure in combination with the surface tension of the liquid that keeps the liquid inside the tube. As long as the surface tension is such that the liquid can form drops larger than the width of the internal diameter D of the tube and the meniscus 3 is convex, which means that the cohesion between the liquid molecules is greater than the adhesion between the liquid molecules and the wall molecules, the liquid tends to flow through the tube as a "whole" and not as a thin layer on the inner side of the tube wall.

A special embodiment of the device according to the invention, by means of which the liquid flow and a liquid dosage can be measured continuously and precisely, is characterized in that the tube is provided with an orifice near its inflow opening, which orifice places the inside of the tube into communication with the outside environment, such that the surface tension of the liquid in the orifice attempts to find an equilibrium with the hydrostatic pressure of the weight of the liquid column that is present under the orifice, and wherein the length of the free liquid column that has formed after the disruption of said equilibrium is a measure of the amount of liquid that has been released.

The length of the liquid column in the tube is limited by using an orifice 4 in the tube wall which has a small diameter in comparison with the diameter D of the tube 1, which orifice places the inside of the tube 1 into communication with the environment. The fact is that the liquid column will break at the location of the orifice 4 when the hydrostatic pressure W (see arrow W in Figure 1a) of the weight of the liquid column hanging under the orifice 4 in the tube 1 is greater than the compensating surface tension T of the liquid (see arrow T in Figure 1a). As a result of the hydrostatic pressure of the weight of the liquid column at the location of orifice 4, a small gas bubble 5 will begin to grow, as is shown in Figure 1a. When the weight becomes too great, the column 2a will break and the liquid the will flow from the tube in the form of a column segment, as is shown in Figure 1b.

The free column segment 2a thus formed has a characteristic length, and thus a volume which can be precisely determined, which length depends on the dimension of the orifice 4 and the interaction between the liquid and the tube at the location of the orifice 4. From this a liquid dosage can easily be derived.

The surface tension of the liquid as well as the dimension of the orifice 4 prevent the liquid from exiting through the orifice 4. This physical phenomenon can be compared with inflating a balloon. Initially it is difficult to inflate the balloon; once a threshold value is exceeded, however, inflation is much easier.

It may be possible to shut off the orifice, for example by means of a valve. If a valve 6 is arranged in front of the orifice 4, as is shown in Figure 1c, it is possible to allow the liquid column to continue to grow. In the closed position of the valve 6, the liquid will flow out in the form of drops 7 or in the form of a jet, depending on the magnitude of the liquid flow, at the outflow opening 1b of the tube 1. The magnitude of the liquid flow to which the invention relates usually leads to drops being formed.

When the surface tension in a small orifice 4 is utilised for causing the column 2 to break without making use of a valve, as is shown in Figure 1d again, the length of the column at which said column breaks can be determined in part on the basis of a changing property of the material of the inner wall. In accordance with the invention, the tube may be provided with a liquid-attracting material on the inner side, near the outflow opening, for the purpose of limiting the liquid column to a specific length.

By applying a material 8 to the inner wall near the outflow opening 1b, which material leads to an adhesion between the liquid molecules and the wall molecules which is greater than the cohesion between the liquid molecules, the liquid is additionally drawn in the direction of the opening 1b, as it were, and the liquid column will thus break at a specific length L (see Figure 1d) with greater precision. This makes it possible to obtain a dosage amount which is easier to reproduce.

The opening of the orifice 4 by means of a valve 6 as well as the utilisation of the liquid-attracting action of the wall material 8 make it possible to achieve that the volume of the liquid column that breaks off will be the same at all times. This known volume enables calibration both of the liquid dosaging method and of the liquid flow measuring method, because the length L and the tube diameter D fully determine the volume of the liquid being released.

When a comparison is made with the prior art devices as described above, in which measurements are carried out on hanging drops, it can be stated that the vibrating drops are "tamed" by the tube. Also in the process of drops growing and falling from an outflow opening, the moment of falling depends on the surface tension and the adhesion of the liquid to the outflow opening. Comparable to EP-0 610 418, the growth of the liquid column is a measure of the liquid flow, and the precise dimension of the length of the column at the point at which the column breaks is not relevant. This measurement still needs to be calibrated, however, with the exact information as regards to the drop size at any, albeit random moment.

Using a liquid-attracting material 8 near the outflow opening 1b in the tube, the calibration volume is determined by the internal diameter of the tube and the length of the tube up to the level of a convex meniscus in the liquid. When a valve 6 is used, it is even possible to adjust and precisely predetermine the length at which the column will break. This self-calibrating characteristic constitutes an advantage over EP-0 610 418, in which the optical magnification factor must be determined. It also constitutes an advantage over US-A-4,936,828, in which the number of falling drops must be counted, whose size can never be exactly determined, however. It also constitutes an advantage over the measuring methods which utilise a riser tube for measuring the liquid flow, in which the amount of liquid that remains behind when the tube is emptied renders the measurement more difficult.

The above-described physical phenomenon, in which the environmental pressure and the surface tension hold the liquid in its position in the tube as long as the surface tension of the liquid in the orifice 4, which is intended is to have the liquid column "drop" or break at that location, is greater than the hydrostatic pressure at that position, which pressure is determined by the mass of the liquid column below the position of the orifice, can be combined with another physical phenomenon, in which the speed at which the surrounding gas flows into the tube is reduced by a gas flow restrictor 10 in combination with the orifice 4. This will be explained with reference to Figure 1e.

If a restrictor 10 is arranged between the orifice 4 and the environment, this will reduce the speed at which the liquid column 11 moves to the outflow opening 1b in the tube, once it has broken off. Said speed does not depend on the speed at which the liquid flows into the tube 1 at the inflow opening 1a as a result of the existence of a liquid flow. The time during which the broken liquid column, which is now a liquid segment 11, moves "freely", on the other hand, does depend on the speed at which the liquid flows. The fact is that the newly grown column blocks the orifice 4 again, and the rate at which that happens depends only on the liquid flow.

With the passage of time, a pattern of liquid segments 11 and gas segments 12 characteristic of the liquid flow is thus formed. The dimensions of the segments are a measure of the liquid flow through the tube. In this element of the invention, a kind of implicit "clock" is combined with the liquid flow proper in the measurement by the flow restrictor 10 for the inflow of gas into the tube 1. The inflow resistance depends on the viscosity of the inflowing gas; if the type of gas does not change it will not be necessary to calibrate the internal "clock" anew each time.

The speed at which a liquid segment 11 moves through the tube can also be reduced by placing a flow restrictor for the liquid near the outflow opening 1b of the tube. In that case, the different viscosity of different liquids makes it difficult to calibrate the "clock", however.

Another possibility of the physical phenomenon, in which the equilibrium in the hydrostatic pressure and the surface tension of the liquid, combined with a liquid- repellent action of the wall material, is utilised for determining the dosage or the liquid flow, shows the use of a tube whose diameter increases in the direction of the outflow opening, in such a manner that the surface tension of the liquid at the bottom of the liquid column attempts to find an equilibrium with the adhesion of the liquid to the tube wall, after which this equilibrium will be disrupted when a specific diameter is reached and liquid can flow off along the wall, until the surface tension of the liquid at a point in the tube above the position of said equilibrium is greater than the cohesion between the liquid and the tube wall, and wherein, after the equilibrium has been disrupted, the free liquid column that has then formed is a measure of the released amount of liquid and measuring of the liquid flow from the new, downwardly growing liquid column is possible again.

Compared with the method that is currently being used by nurses for checking on the liquid flow when administering an infusion, namely the counting of drops in a so-called drip chamber over a specific period of time, a liquid flow measuring method in which the speed of the liquid flow can be checked on at one glance from the length of the segments can have a time-saving effect. On the other hand it is also possible, by making the orifice 4 optional, to continue to count the number of drops per unit time for determining the liquid flow. The only thing that is required in that case is not to remove an optional, removable cover from the orifice.

### Liquid flow measurement by measuring the difference in light intensity

A next element of the invention relates to the injection of the growth of the liquid column. Compared with the method measuring from drops, in which a volumetric value of drops is to be computed on the basis of two-dimensional image information, a one-dimensional measuring method will suffice in a liquid flow measuring method in which a tube is used. After all, the diameter of the tube is known, so that only the length of the liquid column must be determined. This can be realised with a one-dimensional sensor. According to US-A-5,355,735, the sensor may consist of a row of photosensitive cells that detect the position of the meniscus in the liquid. A higher resolution can be achieved with a line CCD as shown in US-A-5,333,497.

According to the invention, the device in which the tube is transparent is characterized in that one sensor is disposed beside the tube, from which electro-magnetic radiation directed at the tube and the liquid column is reflected, the reflection being a measure of the length of the liquid column in the tube. Comparable to EP-0 610 418, it is not the current position of the meniscus that is important but the increase of the length of the column. Preferably, the relation between the length of the liquid column and the measured light intensity is proportional. When the tube in which the liquid column is present is irradiated with light in a direction parallel to the axis of the tube, said light will reflect from the boundary layer defined by the tube material and the gas in the tube as a result of the smaller optical refractive index of said gas. When the boundary layer is defined by the tube material and the liquid, the light rays will be broken or adsorbed. According to British patent No 1,426,824, the contrast that is achieved with the reflection method is higher than the contrast that is achieved with the method in which the light source is disposed diametrically opposite the sensor on the other side of the tube, in which the broken light rays provide the contrast required for determining the position of the meniscus. The problem of inhomogeneities in the light gap that is used in GB-1,426,824 is remedied in US-A-5,333,497 through the use of a diffuser in the light gap.

The main advantage of the use of reflective rays, however, is the fact that the relation between the length of the column and the amount of light that is reflected is proportional.

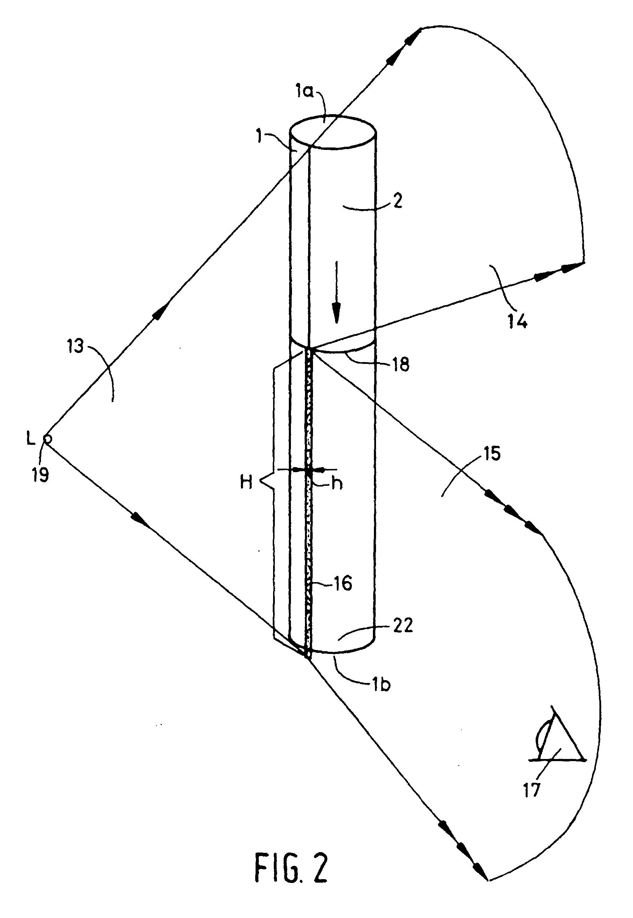

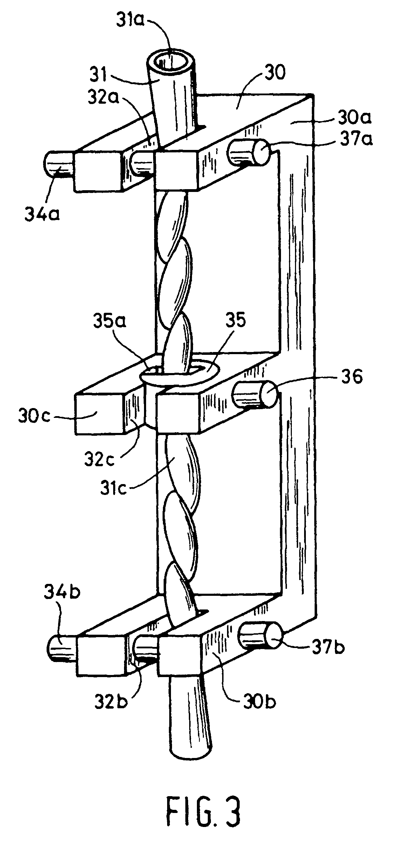

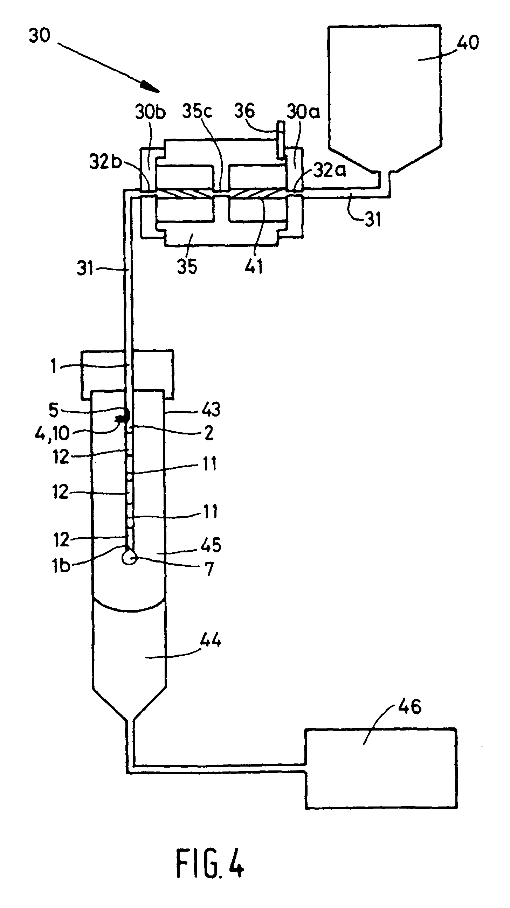

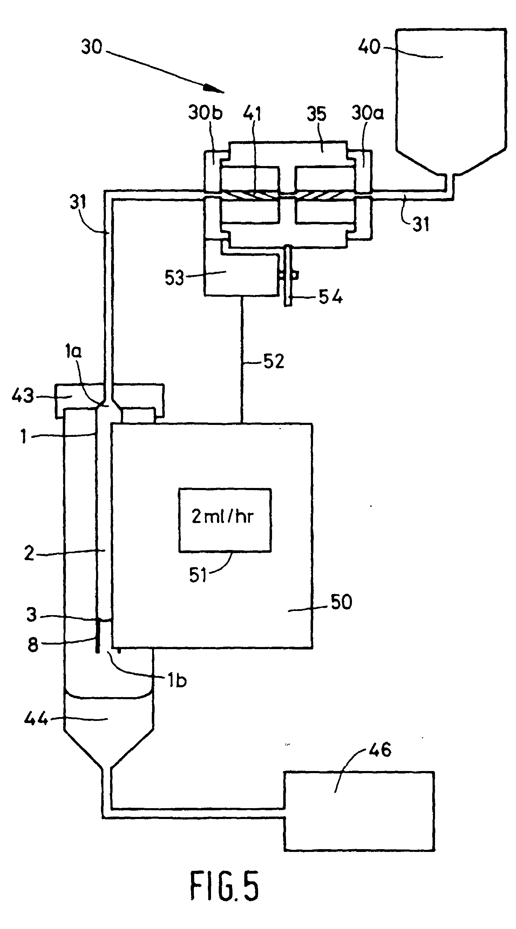

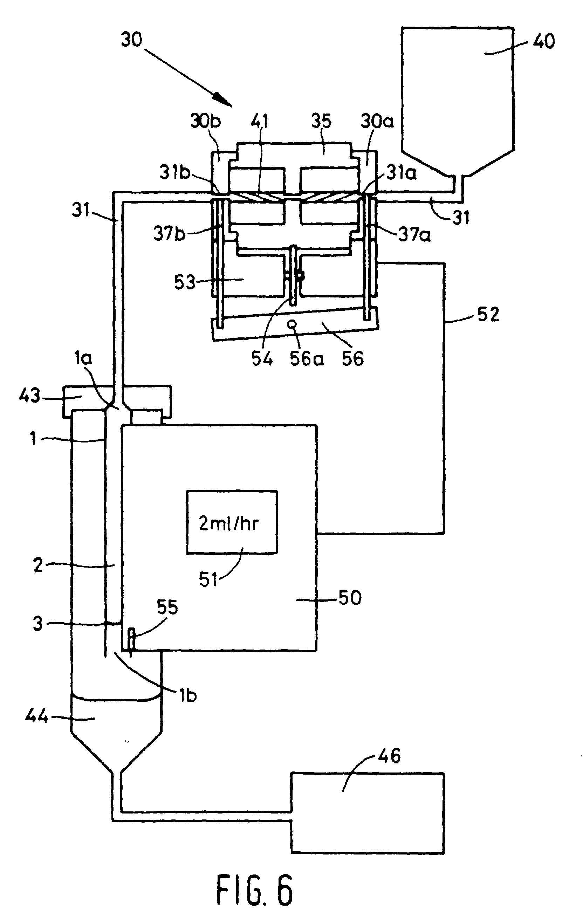

With transparency-based systems, the determination of the position of the meniscus is rendered more difficult by the parallax of the width of the tube. Different parts of the meniscus absorb the light rays in dependence on the position of the meniscus relative to the position of the sensor. In addition to that, the meniscus reflects a different amount of light with each change of position. When the reflection at the transition between the tube and the gas is used, the same part of the meniscus is used at all times for determining the transition from gas to liquid, as is shown in Figure 2.

By making use of the reflection, the length of the liquid column can therefore be measured by means of a single sensor 17, which measures the radiation intensity, and wherein a single radiation point source 19 (L) rather than a line source may irradiate the tube 1. The upper electro-magnetic rays 13 propagate straight ahead (possibly broken) as transmitted radiation 14 in the liquid-filled part 2 of the tube 1. The lower rays 15 are reflected from the transition 16 between the tube material and the gas in the part 22 of the tube that is not filled with liquid yet, in which the sensor 17 detects the overall intensity of the reflective rays that fall within its range of vision. Owing to the cylindrical mirror optics of the curved tube wall, a reflected light line 16 whose width h depends on the curvature parallel to the axis of the tube and the position of the point source 19 relative to the sensor 17 is formed upon irradiation of the tube. Since said width h remains constant within the range of the length of the liquid column, only the length H of the light line 16 is a determining factor as regards the progress of the meniscus 18 in the direction of the outflow opening 1b.

### Other applications

The invention as described above can be used in many kinds of apparatuses, such as: liquid flow meters for testing other systems, apparatuses for automatically metering the amount of syrup in soft drink dispensers, apparatuses for monitoring the urine production of patience and apparatuses for dosaging reacting agents in systems in which pipettes are generally used.

## Claims

1. A device for measuring a liquid flow (2) through a tube (1), which tube has an inflow side (1a) and an outflow side (1b) disposed under said inflow side, and is filled with said liquid (2), as a result of which a liquid column is formed in the tube, wherein the tube is provided with an orifice (4) near its inflow opening (1a), which orifice (4) places the inside of the tube into communication with the outside environment, such that the surface tension of the liquid in the orifice (4) tries to find an equilibrium with the hydrostatic pressure of the weight of the liquid column (2a) that is present under the orifice, and wherein the length of the free liquid column (11) that has formed after the disruption of said equilibrium is a measure of the amount of liquid that has been released, **characterized in that** said orifice (4) is provided with a restrictor (10) for regulating the inflow of gas from the outside environment after the equilibrium has been disrupted.

2. A device according to claim 1, **characterized in that**, said restrictor (10) is constructed as a valve (6).

3. A device according to claim 1 or 2, **characterized in that** the inner side of the tube is provided with a liquid-attracting material (8) near its outflow opening (1b) for the purpose of limiting the liquid column (12) to a specific length.

4. A device according to any one or more of the preceding claims, **characterized in that,** the tube diameter increases in the direction of the outflow opening, in such a manner that the surface tension of the liquid at the bottom of the liquid column attempts to find an equilibrium with the adhesion of the liquid to the tube wall, after which this equilibrium will be disrupted when a specific diameter is reached and liquid can flow off along the wall, until the surface tension of the liquid at a point in the tube above the position of said equilibrium is greater than the cohesion between the liquid and the tube wall, and wherein, after the equilibrium has been disrupted, the free liquid column that has then formed is a measure of the released amount of liquid and measuring of the liquid flow from the new, downwardly growing liquid column is possible again.

5. A device according to any one or more of the preceding claims, wherein the tube is transparent, **characterized in that** one sensor is disposed beside the tube, on which electro-magnetic radiation directed at the tube and the liquid column is reflected, the reflection being a measure of the length of the liquid column in the tube.

## Patentansprüche

1. Vorrichtung zur Messung einer Flüssigkeitsströmung (2) durch ein Rohr (1), wobei das Rohr eine Zuflussseite (1a) und eine unterhalb der Zuflussseite angeordnete Ausflussseite (1b) aufweist und mit der Flüssigkeit (2) gefüllt ist, wodurch eine Flüssigkeitssäule im Rohr gebildet ist, wobei das Rohr mit einer Öffnung (4) in der Nähe der Zuflussöffnung (1a) versehen ist, und diese Öffnung (4) das Innere des Rohres auf eine solche Weise in Verbindung mit der äußeren Umgebung bringt, dass die Oberflächenspannung der Flüssigkeit in der Öffnung (4) versucht, ein Gleichgewicht mit dem hydrostatischen Druck des Gewichts der Flüssigkeitssäule (2a) zu finden, die unterhalb der Öffnung vorhanden ist, und wobei die Länge der freien Flüssigkeitssäule (11), die sich nach der Zerstörung des Gleichgewichts gebildet hat, ein Maß für die Flüssigkeitsmenge darstellt, die freigesetzt worden ist, **dadurch gekennzeichnet, dass** die Öffnung (4) mit einem Reduzierer (10) versehen ist, um den Zufluss von Gas aus der äußeren Umgebung zu regulieren, nachdem das Gleichgewicht zerstört worden ist.

2. Vorrichtung nach Anspruch 1, **dadurch gekennzeichnet, dass** der Reduzierer (10) als Ventil (6) ausgebildet ist.

3. Vorrichtung nach Anspruch 1 oder 2, **dadurch gekennzeichnet, dass** die Innenseite des Rohres nahe der Ausflussöffnung (1 b) mit einem flüssigkeitsanziehenden Material (8) versehen ist, um die Flüssigkeitssäule (12) auf eine bestimmte Länge zu begrenzen.

4. Vorrichtung nach einem oder mehreren der vorhergehenden Ansprüche, **dadurch gekennzeichnet, dass** sich der Rohrdurchmesser in Richtung der Ausflussöffnung auf eine solche Weise vergrößert, dass die Oberflächenspannung der Flüssigkeit an der Unterseite der Flüssigkeitssäule versucht, ein Gleichgewicht mit der Adhäsion der Flüssigkeit an der Rohrwand zu finden, wonach dieses Gleichgewicht dann zerstört wird, wenn ein bestimmter Durchmesser erreicht ist, und Flüssigkeit entlang der Wand abfließen kann, bis die Oberflächenspannung der Flüssigkeit an einem Punkt im Rohr oberhalb der Position dieses Gleichgewichts größer ist, als die Kohäsion zwischen der Flüssigkeit und der Rohrwand, und wobei, nachdem das Gleichgewicht zerstört worden ist, die freie Flüssigkeitssäule, die sich gebildet hat, ein Maß für die freigesetzte Flüssigkeitsmenge ist, und das Messen der Flüssigkeitsströmung von der neuen, nach unten wachsenden Flüssigkeitssäule neuerlich möglich ist.

5. Vorrichtung nach einem oder mehreren der vorhergehenden Ansprüche, wobei das Rohr durchsichtig ist, **dadurch gekennzeichnet, dass** ein Sensor neben dem Rohr angeordnet ist, auf dem auf das Rohr und die Flüssigkeitssäule gerichtete elektromagnetische Strahlung reflektiert wird, wobei die Reflexion ein Maß für die Länge der Flüssigkeitssäule im Rohr ist.

## Revendications

1. Dispositif de mesure d'un débit de liquide (2) à travers un tube (1), ledit tube ayant un côté d'admission (1a) et un côté d'écoulement (1 b) disposé en-dessous dudit côté d'admission, et étant rempli avec ledit liquide (2) de sorte qu'une colonne de liquide se forme dans le tube, dans lequel le tube est pourvu d'un orifice (4) au voisinage de son ouverture d'admission (1a), ledit orifice (4) mettant l'intérieur du tube en communication avec l'environnement extérieur, de sorte que la tension superficielle du liquide dans l'orifice (4) tend à établir un équilibre avec la pression hydrostatique due au poids de la colonne de liquide (2a) qui est présente en-dessous de l'orifice, et dans lequel la hauteur de la colonne de liquide libre (11) qui s'est formée après la rupture dudit équilibre constitue une mesure de la quantité de liquide ayant été relâché, **caractérisé en ce que** ledit orifice (4) est pourvu d'un limiteur (10) pour réguler l'admission de gaz depuis l'environnement extérieur après que l'équilibre ait été rompu.

2. Dispositif selon la revendication 1, **caractérisé en ce que** la construction dudit limiteur (10) a la forme d'une soupape (6).

3. Dispositif selon la revendication 1 ou 2, **caractérisé en ce que** la face interne du tube est pourvue d'un revêtement (8) attirant le liquide, situé au voisinage de son orifice d'écoulement (1b) aux fins de limiter la colonne de liquide (12) à une hauteur définie.

4. Dispositif selon l'une quelconque ou plusieurs des revendications précédentes, **caractérisé en ce que** le diamètre du tube augmente dans la direction de l'ouverture d'écoulement, de telle sorte que la tension superficielle du liquide en bas de la colonne de liquide tend à établir un équilibre avec la force d'adhérence du liquide à la paroi du tube, après quoi cet équilibre est rompu lorsqu'un diamètre prédéfini est atteint et le liquide peut s'écouler le long de la paroi, jusqu'à ce que la tension superficielle du liquide en un point dans le tube situé au-dessus de la position dudit équilibre soit supérieure à la force de cohésion entre le liquide et la paroi du tube, et dans lequel, après que l'équilibre ait été rompu, la colonne de liquide libre qui s'est alors formée est une mesure de la quantité de liquide relâché et la mesure du débit de liquide pour la nouvelle colonne croissant vers le bas est à nouveau possible.

5. Dispositif selon l'une quelconque ou plusieurs des revendications précédentes, dans lequel le tube est transparent, **caractérisé en ce qu'**un capteur est disposé à proximité du tube, sur lequel un rayonnement électromagnétique dirigé vers le tube est réfléchi, ladite réflexion constituant une mesure de la hauteur de la colonne de liquide dans le tube.
